# EUROPEAN PATENT SPECIFICATION

(11) **EP 2 145 583 B1**
(45) Date of publication and mention of the grant of the patent: **24.06.2015**
(21) Application number: 09164404.7
(22) Date of filing: 02.07.2009
(51) Int. Cl.: A61B 8/08

(54) **Formation of an elastic image using an ultrasound apparatus and method thereof**
Bildung eines elastischen Bildes in einer Ultraschallvorrichtung und das entsprechende Verfahren
Formation d'une image élastique à l'aide d'un appareil à ultrasons, ainsi que la methode correspondante

(30) Priority: 16.07.2008 KR 20080068909
(43) Date of publication of application: 20.01.2010
(73) Proprietor: Samsung Medison Co., Ltd., Hongcheon-gun, Gangwon-do (KR)
(72) Inventor: Kim, Jong Sik, 135-851 Seoul (KR); Shin, Dong Kuk, 135-851 Seoul (KR)
(74) Representative: Schmid, Wolfgang

(56) References cited:
- EP-A1- 1 554 982
- EP-A1- 1 591 068
- EP-A1- 1 938 754
- TSUZUKI ET AL: "Optimal Region-of-Interest Settings for Tissue Characterization Based on Ultrasonic Elasticity Imaging" ULTRASOUND IN MEDICINE AND BIOLOGY, NEW YORK, NY, US, vol. 34, no. 4, 9 January 2008 (2008-01-09), pages 573-585, XP022547226 ISSN: 0301-5629

## Description

### TECHNICAL FIELD

The present disclosure generally relates to ultrasound systems, and more particularly to the formation of an elastic image in an ultrasound system.

### BACKGROUND

Recently, an ultrasound system has been extensively used in the medical field due to its non-invasive and non-destructive nature. Modem high-performance ultrasound imaging systems and techniques are commonly used to produce two dimensional ultrasound images and three-dimensional ultrasound images of internal features of patients.

Generally, the ultrasound image is displayed in a Brightness mode (B-mode) by using reflectivity caused by an acoustic impedance difference between the tissues of a target object. However, if the reflectivity of the target object is hardly different from those of the neighboring tissues such as tumor, cancer or the like, then it is not easy to recognize the target object in the B-mode image. Further, an ultrasound elastic imaging technology has been developed to display an image of the target object by using mechanical characteristics of the target object. Such technology is very helpful for diagnosing lesions such has cancers. The tumor or cancer is relatively stiffer than the neighboring tissue. Thus, when pressure is uniformly applied, a variation of the tumor or cancer is typically smaller than those of the neighboring tissues.

The elasticity of a tissue is measured by using ultrasound data obtained before and after application of the pressure to the tissue. A compression plate mounted on an ultrasound probe may be used to apply the pressure to the tissue. A user may press the compression plate on the target object, thereby applying the pressure to the tissues of the target object. In such a case, strain data in the tissues may be varied according to the pressure applied by the user. Thus, the video quality of an elastic image may be changed according two the pressure applied to the tissue.

EP 1 938 754 A1 discloses an ultrasound system according to the preamble of claim 1 and a method of forming an elastic image in an ultrasound system according to the preamble of claim 3.

EP 1 591 068 A1 discloses an ultrasonographic device comprising an ultrasound probe including an oscillator for generating an ultrasonic wave, ultrasonic wave transmitter/receiver means connected to the probe, phasing addition means which controls a phrase of the received ultrasonic wave and generates RF signal frame data, an RF signal frame data selection section for making variable a frame interval of the RF signal frame data, an elastic frame data calculation section for generating plastic frame data in time sequence based on a pair of the RF signal frame data and an elastic image generating section for generating an elastic image based on the plastic frame data.

EP 1 554 982 A1 discloses an ultrasound diagnostic apparatus which includes means for generating a tomographic image from a reflected echo signal received by the probe corresponding to an ultrasound wave transmitted by a probe put on an object to be examined, means for measuring a displacement of the object's tissue and calculating elasticity information on the basis of a reflected echo signal received from the object corresponding to an ultrasound wave transmitted while the pressure applied when the probe is put on the object is changed, means for generating a translucent image from the color elasticity image on the basis of image information of either the tomographic image or the color elasticity image and means for displaying the combined image.

### SUMMARY

Embodiments for forming an elastic image in an ultrasound system are disclosed herein. In one embodiment, an ultrasound system comprises the features stated in claim 1.

In another embodiment, there is provided a method of forming an elastic image in an ultrasoundsystem according to claim 3.

### BRIEF DESCRIPTION OF THE DRAWINGS

- FIG. 1: is a block diagram showing an illustrative embodiment of an ultrasound system.
- FIG. 2: is a block diagram showing an illustrative embodiment of an ultrasound data acquisition unit.
- FIG. 3: is a block diagram showing an illustrative embodiment of a processing unit.
- FIG. 4: is a schematic diagram showing an example of a histogram.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

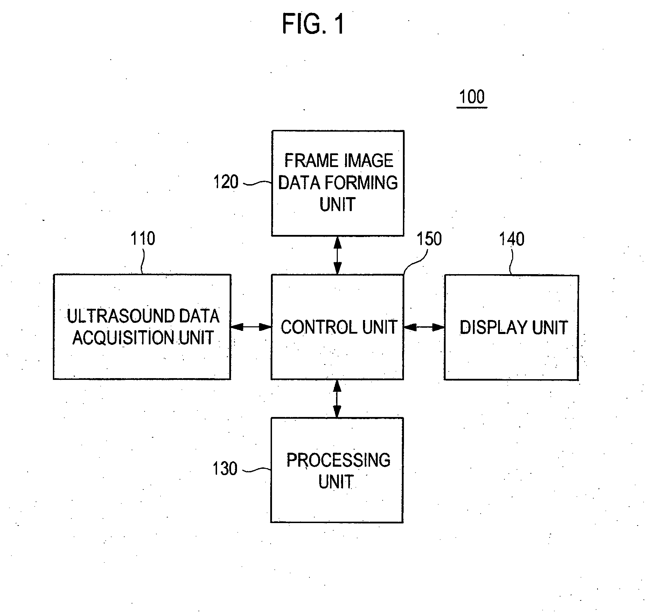

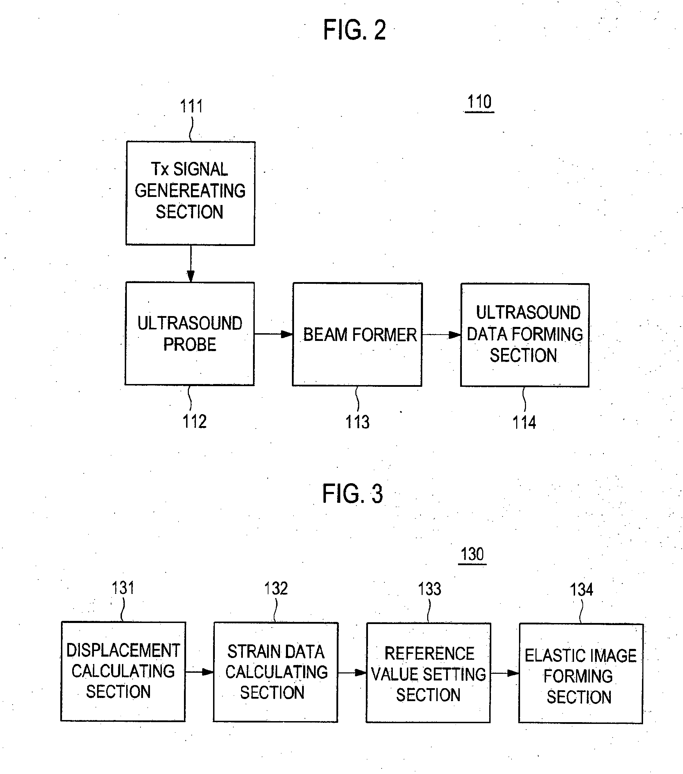

Referring to FIG. 1, an ultrasound system 100 in accordance with an illustrative embodiment is shown. As depicted therein, the ultrasound system 100 may include an ultrasound data acquisition unit 110. The ultrasound data acquisition unit 110 may be operable to transmit/receive ultrasound signals to/from a target object to thereby output ultrasound data. The ultrasound data acquisition unit 110 may include a transmit (Tx) signal generating section 111, as shown in FIG. 2.

Referring to FIG. 2, the Tx signal generating section 111 may be operable to generate first Tx signals before applying pressure to the target object. The Tx signal generating section 111 may be further operable to generate second Tx signals while applying the pressure to the target object.

The ultrasound data acquisition unit 110 may further include an ultrasound probe 112 containing a plurality of elements for reciprocally converting between ultrasound signals and electrical signals. The ultrasound probe 112 may be operable to transmit ultrasound signals into the target object in response to the first Tx signals. The ultrasound probe 112 may be further operable to receive echo signals reflected from the target object to thereby output first received signals. The received signals may be analog signals. The ultrasound probe 112 may be further operable to transmit ultrasound signals into the target object in response to the second Tx signals. The ultrasound probe 112 may be further operable to receive echo signals reflected from the target object to thereby output second received signals.

The ultrasound data acquisition unit 110 may further include a beam former 113. The beam former 113 may be operable to convert the first received signals into first digital signals. The beam former 113 may be further operable to apply delays to the first digital signals in consideration of distance between the elements and focal points to thereby output first digital receive-focused signals. The beam former 113 may be further operable to convert the second received signals into second digital signals.
The beam former 113 may be further operable to apply delays to the second digital signals in consideration of distance between the elements and focal points to thereby output second digital receive-focused signals.

The ultrasound data acquisition unit 110 may further include an ultrasound data forming section 114. The ultrasound data forming section 114 may be operable to form first ultrasound data based on the first digital receive-focused signals. The ultrasound data forming section 114 may be further operable to form second ultrasound data based on the second digital receive-focused signals.

Referring back to FIG. 1, the ultrasound system 100 may further include a frame image data forming unit 120 that may be operable to form frame image data. The frame image may include a brightness mode (B-mode) image represented with gray scales based on reflectivity of the ultrasound signals in the target object. The frame image data forming unit 120 may be operable to form first frame image data of a first frame image based on the first ultrasound data. The frame image data forming unit 120 may be further operable to form second frame image data of a second frame image based on the second ultrasound data.

The ultrasound system 100 may further include a processing unit 130 that may be operable to form an elastic image. The processing unit 130 may include a displacement calculating section 131, as shown in FIG. 3.

Referring to FIG. 3, the displacement calculating section 131 may be operable to calculate displacements between the first and second frame image data. The displacement may be calculated on pixel-by-pixel or block-by-block basis between the first and second frame images by using the first and second frame image data. Also, the displacement may be calculated by using a cross-correlation or an auto-correlation.

The processing unit 130 may further include a strain data calculating section 132. The strain data calculating section 132 may be operable to calculate strain data based on the displacements.

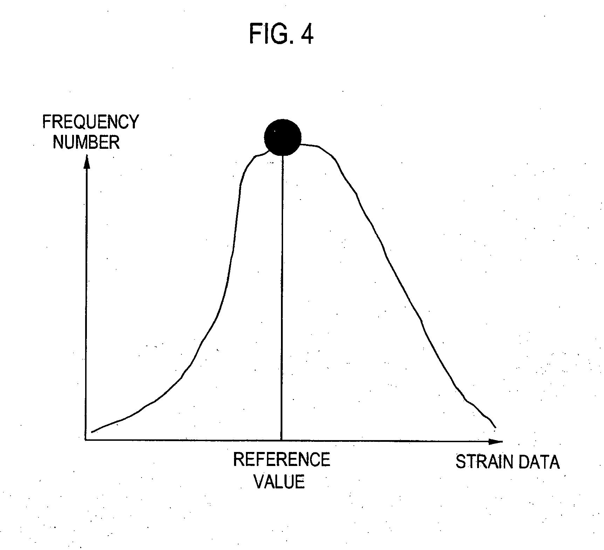

The processing unit 130 may further include a reference value setting section 133 that may be operable to set a reference value. The reference value may include one of a maximum value of frequency numbers of numerical values of the strain data and a mean value of the numerical values of the strain data. In one embodiment, the reference value setting section 133 may be operable to normalize the strain data to thereby form a histogram by using the strain data, as shown in FIG. 4. The reference value setting section 133 may be further operable to analyze the histogram to thereby detect a maximum value of frequency numbers of the numerical values of the strain data. The reference value setting section 133 may be operable to set the detected maximum value as the reference value. In one embodiment, the reference value setting section 133 may be operable to calculate a mean value of the numerical values of the strain data. The reference value setting section 133 may be further operable to set the mean value as the reference value.

The processing unit 130 may further include an elastic image forming section 134.
The elastic image forming section 134 may be operable to form an elastic image based on the reference value. In one embodiment, the elastic image forming section 134 may be operable to detect strain data that are less than the reference value. The elastic image forming section 134 may be further operable to form the elastic image based on the detected strain data. The elastic image may be an elastic image of a stiff tissue (e.g., lesion). In one embodiment, the elastic image forming section 134 may be operable to detect strain data that are greater than the reference value. The elastic image forming section 134 may be further operable to form the elastic image based on the detected strain data. The elastic image may be an elastic image of a soft tissue. In one embodiment, the elastic image forming section 134 may be operable to detect strain data that are less and greater than the reference value. The elastic image forming section 134 may be further operable to form the elastic image based on the detected strain data.

Referring back to FIG. 1, the ultrasound system 100 may further include a display unit 140. The display unit 140 may be operable to display the elastic image formed in the processing unit 130.

The ultrasound system 100 may further include a control unit 150. The control unit 150 may be operable to control the transmission/reception of the ultrasound signals and formation of the ultrasound data in the ultrasound data acquisition unit 110. The control unit 150 may be further operable to control the formation of first and second frame image data in the frame image data forming unit 120. The control unit 150 may be further operable to control the formation of the elastic image in the processing unit 130. The control unit 150 may be further operable to control the display of the elastic image.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system (150), comprising:
an ultrasound data acquisition unit (110) configured to transmit/receive ultrasound signals to/from a target object to thereby output first ultrasound data and to transmit/receive ultrasound signals to/from the target object while applying a pressure to the target object to thereby output second ultrasound data;
a frame image data forming unit (120) configured to form a first frame image data based on the first ultrasound data and to form a second frame image data based on the second ultrasound data;
a processing unit (130) configured to calculate strain data based on the first and second frame image data; and
a control unit (150) coupled to the ultrasound data acquisition unit (110), the frame image data forming unit (120) and the processing unit (130), the control unit being (150) configured to control transmission/reception of the ultrasound signals and formation of the first and second frame image data,
wherein the processing unit (130) is further configured to detect a maximum value of frequency numbers of numerical values of the strain data, to set the maximum value as reference value, to detect strain data less than the reference value or strain data greater than the reference value to form an elastic image and to form an elastic image based on the detected strain data.

2. The ultrasound system (100) of Claim 1, wherein the processing unit comprises:
a displacement calculating section (131) configured to calculate displacements between the first and second frame image data;
a strain data calculating section (132) configured to calculate the strain data based on the displacements;
a reference value setting section (133) configured to set the reference value based on the strain data; and
an elastic image forming section (134) configured to form the elastic image based on the reference value.

3. A method of forming an elastic image in an ultrasound system (100), comprising:
a) transmitting/receiving by using an ultrasound data acquisition unit (110) within the ultrasound system (100) ultrasound signals to/from a target object to thereby output first ultrasound data;
b) forming by using a processing unit (130) within the ultrasound system (100) a first frame image data based on the first ultrasound data;
c) transmitting/receiving by using the ultrasound data acquisition unit (110) within the ultrasound system (100) ultrasound signals to/from the target object while applying pressure to the target object to thereby output second ultrasound data;
d) forming by using the processing unit (130) within the ultrasound system (100) a second frame image data based on the second ultrasound data; and
e) calculating by using the processing unit (130) within the ultrasound system (100) strain data based on the first and second frame data,
f) detecting a maximum value of frequency numbers of numerical values of the strain data
g) setting the maximum value as a reference value,
h) detecting strain data less than the reference value or strain data greater than the reference value to form the elastic image
i) forming the elastic image based on the detected strain data.

4. The method of Claim 3, wherein the step e) comprises:
e1) calculating displacements between the first and second frame image data;
e2) calculating the strain data based on the displacements;

## Patentansprüche

1. Ultraschallsystem (100), welches Folgendes aufweist:
eine Ultraschalldatenerlangungseinheit (110), die dafür vorgesehen ist, Ultraschallsignale zu bzw. von einem Zielobjekt zu übertragen bzw. zu empfangen,
um dadurch erste Ultraschalldaten auszugeben, und zu bzw. von dem Zielobjekt Ultraschallsignale zu übertragen bzw. zu empfangen, während ein Druck auf das Zielobjekt aufgebracht wird, um dadurch zweite Ultraschalldaten auszugeben;
eine Rahmenbilddatenerzeugungseinheit (120), die dafür vorgesehen ist, erste Rahmenbilddaten basierend auf den ersten Ultraschalldaten zu erzeugen und
zweite Rahmenbilddaten basierend auf den zweiten Ultraschalldaten zu erzeugen;
eine Verarbeitungseinheit (130), die dafür vorgesehen ist, Spannungsdaten basierend auf den ersten und zweiten Rahmenbilddaten zu berechnen; und
eine Steuereinheit (150), die mit der Ultraschalldatenerlangungseinheit (110),
der Rahmenbilddatenerzeugungseinheit (120) und der Verarbeitungseinheit (130) verbunden ist, wobei die Steuereinheit (150) dafür vorgesehen ist, die Übertragung bzw. den Empfang der Ultraschallsignale und die Erzeugung der ersten und zweiten Rahmenbilddaten zu steuern,
wobei die Verarbeitungseinheit (130) des Weiteren dafür vorgesehen ist, einen Maximalwert von Frequenz- bzw. Häufigkeitswerten von numerischen Werten der Spannungsdaten zu detektieren, den Maximalwert als Referenzwert festzulegen, Spannungsdaten, die geringer sind als der Referenzwert, oder Spannungsdaten, die größer sind als der Referenzwert, zu detektieren, um ein elastisches Bild zu erzeugen, und ein elastisches Bild basierend auf den detektierten Spannungsdaten zu bilden.

2. Ultraschallsystem (100) nach Anspruch 1, wobei die Verarbeitungseinheit Folgendes aufweist:
einen Verschiebungsberechnungsabschnitt (131), der dafür vorgesehen ist,
Verschiebungen zwischen den ersten und zweiten Rahmenbilddaten zu berechnen;
einen Spannungsdatenberechnungsabschnitt (132), der dafür vorgesehen ist,
die Spannungsdaten basierend auf den Verschiebungen zu berechnen;
einen Referenzwertfestlegungsabschnitt (133), der dafür vorgesehen ist, den Referenzwert basierend auf den Spannungsdaten festzulegen, und
einen Erzeugungsabschnitt (134) für ein elastisches Bild, der dafür vorgesehen ist, das elastische Bild basierend auf dem Referenzwert zu erzeugen.

3. Verfahren zur Erzeugung eines elastischen Bilds in einem Ultraschallsystem (100), welches Folgendes aufweist:
a) Übertragen bzw. Empfangen von Ultraschallsignalen zu bzw. von einem Zielobjekt unter Verwendung einer Ultraschalldatenerlangungseinheit (110) innerhalb des Ultraschallsystems (100), um dadurch erste Ultraschalldaten auszugeben;
b) Erzeugen von ersten Rahmenbilddaten basierend auf den ersten Ultraschalldaten unter Verwendung einer Verarbeitungseinheit (130) innerhalb des Ultraschallsystems (100);
c) Übertragen bzw. Empfangen von Ultraschallsignalen zu bzw. von dem Zielobjekt unter Verwendung der Ultraschalldatenerlangungseinheit (110) innerhalb des Ultraschallsystems (100), während ein Druck auf das Zielobjekt aufgebracht wird, um dadurch zweite Ultraschalldaten auszugeben;
d) Erzeugen von zweiten Rahmenbilddaten basierend auf den zweiten Ultraschalldaten unter Verwendung der Verarbeitungseinheit (130) innerhalb des Ultraschallsystems (100); und
e) Berechnen von Spannungsdaten basierend auf den ersten und zweiten Rahmendaten unter Verwendung der Verarbeitungseinheit (130) innerhalb des Ultraschallsystems (100),
f) Detektieren eines Maximalwerts von Frequenz- bzw. Häufigkeitswerten von numerischen Werten der Spannungsdaten;
g) Festlegen des maximalen Werts als ein Referenzwert;
h) Detektieren von Spannungsdaten, die geringer sind als der Referenzwert, oder Spannungsdaten, die größer sind als der Referenzwert, um das elastische Bild zu erzeugen;
i) Erzeugen des elastischen Bilds basierend auf den detektierten Spannungsdaten.

4. Verfahren nach Anspruch 3, wobei der Schritt e) Folgendes aufweist:
e1) Berechnen von Verschiebungen zwischen den ersten und zweiten Rahmenbilddaten;
e2) Berechnen der Spannungsdaten basierend auf den Verschiebungen.

## Revendications

1. Système ultrasonique (100) comportant:
une unité (110) d'acquisition de données ultrasoniques agencée pour transmettre/recevoir des signaux ultrasoniques vers/provenant d'un objet cible pour ainsi émettre des premières données ultrasoniques et pour transmettre/recevoir des signaux ultrasoniques vers/provenant de l'objet cible tout en appliquant une pression sur l'objet cible pour ainsi émettre des secondes données ultrasoniques;
une unité (120) de formation d'une image cadre, configurée pour former de premières données d'image cadre sur la base des premières données ultrasoniques et pour former des secondes données d'image cadre sur la base des secondes données ultrasoniques;
une unité de traitement (130) agencée pour calculer des données de contrainte basées sur les premières et les secondes données d'image cadre ; et
une unité de commande (150) connectée à l'unité (110) d'acquisition de données ultrasoniques, à l'unité (120) de formation d'une image cadre et à l'unité de traitement (130), l'unité de commande (150) étant configurée pour commander la transmission/réception des signaux ultrasoniques et la formation des premières et secondes données d'image cadre,
dans lequel l'unité de traitement (130), est en outre configurée pour détecter une valeur maximale de nombre de fréquences de valeurs numériques de données de contraintes, pour fixer la valeur maximale comme référence pour détecter les données de contraintes inférieures à la valeur de référence ou les données de contraintes de référence supérieures à la valeur de référence, pour former une image élastique et pour former une image élastique basée sur les données de contraintes détectées.

2. Système ultrasonique (100) selon la revendication 1, dans lequel l'unité de traitement comprend:
une section (131) de calcul de déplacements configurée pour calculer les déplacements entre les premières et les secondes données d'images cadre;
une section (132) de calcul des données de contraintes configurée pour calculer les données de contraintes basées sur les déplacements;
une section (133) de fixation des valeurs de références, configurée pour fixer les valeurs de références basées sur les données de contraintes; et
une section (134) de formation d'image élastique configurée pour former une image élastique basée sur la valeur de référence.

3. Procédé de formation d'une image élastique dans un système ultrasonique (100) comportant:
a) la transmission/réception, en utilisant une unité d'acquisition de données ultrasonique (110) dans le système ultrasonique (100), de signaux ultrasoniques vers/provenant d'un objet cible pour ainsi émettre des premières données ultrasoniques;
b) la formation par l'utilisation d'une unité de traitement (130) dans le système ultrasonique (100), de premières données d'image cadre sur la base des premières données ultrasoniques;
c) la transmission/réception, en utilisant l'unité d'acquisition de données ultrasonique (110) dans le système ultrasonique (100), de signaux ultrasoniques vers/provenant d'un objet cible en appliquant une pression sur l'objet cible pour ainsi émettre des secondes données ultrasoniques;
d) la formation, en utilisant l'unité de traitement (130), dans le système ultrasonique (100), de secondes données d'image cadre, basées sur les secondes données ultrasoniques ; et
e) le calcul, en utilisant l'unité de traitement (130) dans le système ultrasonique (100), des données de contraintes basées sur les premières et les secondes données cadre,
f) la détection d'une valeur maximale de nombre de fréquences de valeurs numériques de données de contraintes,
g) la fixation de la valeur maximale comme une valeur de référence,
h) la détection des données de contraintes inférieures à la valeur de contrainte de référence inférieure à la valeur de référence ou des données de contraintes supérieures à la valeur de contrainte de référence inférieure à la valeur de référence pour former une image élastique,
i) la formation de l'image élastique basée sur les données de contraintes détectées.

4. Procédé selon la revendication 3, dans lequel l'étape e) comprend :
e1) le calcul des déplacements entre les premières et les secondes données d'images cadre;
e2) le calcul des données de contraintes basées sur les déplacements.
